# EUROPÄISCHE PATENTANMELDUNG

(11) **EP 4 607 531 A1**
(43) Veröffentlichungstag der Anmeldung: **27.08.2025**
(21) Anmeldenummer: 24159210.4
(22) Anmeldetag: 22.02.2024
(51) Int. Cl.: G16H 40/40, G06Q 10/10, G06Q 10/20

(54) **SYSTEM UND ZUGEHÖRIGES VERFAHREN ZUR SOFORTIGEN RISIKOMINDERUNG FÜR MEDIZINPRODUKTE, DIE VON EINER FIELD SAFETY NOTICE (FSN) BETROFFEN SIND**

(71) Anmelder: B. Braun Melsungen AG, 34212 Melsungen (DE)
(72) Erfinder: Reindl, Michael, 34212 Melsungen (DE)
(74) Vertreter: Tergau & Walkenhorst Intellectual Property GmbH

(57) **Zusammenfassung**

System (20) zur sofortigen Risikominderung für medizinische Geräte (22), die von einer FSN betroffen sind, umfassend
- ein FSN-Modul (54), welches eine Datenbank aufweist, in welcher wenigstens eine FSN und eindeutige Kennungen von medizinischen Geräten (22), die von der jeweiligen FSN betroffen sind, sowie ein Katalog von Daten zu Maßnahmen zur sofortigen Risikominderung für die jeweilige FSN gespeichert sind und/oder speicherbar sind;
- ein Geräteverwaltungsmodul (58), in welchem eine Liste von medizinischen Geräten (22) im Einsatz und jeweils die eindeutige Kennung des jeweiligen medizinischen Geräts (22) hinterlegt ist, und in welchem eine Anzahl von Geräteverwaltungsfunktionen implementiert ist;
- wenigstens ein medizinisches Gerät (22), welches eine eindeutige Kennung aufweist, und eine Steuereinheit (32) und eine Anzeigeeinheit (36) umfasst, wobei

• das FSN-Modul (54) dazu ausgebildet ist, digitale Nachrichten mit dem Geräteverwaltungsmodul (58) und/oder mit dem jeweiligen medizinischen Gerät (22), insbesondere über ein Datennetz, auszutauschen, und wobei
• das Geräteverwaltungsmodul (58) dazu ausgebildet ist, digitale Nachrichten mit dem FSN-Modul (54) und/oder mit dem jeweiligen medizinischen Gerät (22), insbesondere über ein Datennetz, auszutauschen, und wobei
• das jeweilige medizinische Gerät (22) dazu ausgebildet ist, digitale Nachrichten mit dem FSN-Modul (54) und/oder mit dem Geräteverwaltungsmodul (58), insbesondere über ein Datennetz, auszutauschen und Maßnahmen zur sofortigen Risikominderung durchzuführen.

## Beschreibung

Die Erfindung betrifft ein System und ein zugehöriges Verfahren zur sofortigen Risikominderung für Medizinprodukte, die von einer Field Safety Notice (FSN) betroffen sind.

Verfahren aus dem Stand der Technik für die Ausführung eines FSN für ein Medizinprodukt bzw. medizinisches Gerät können durch die folgenden Schritte charakterisiert werden:
1. Starten einer neuen FSN
2. Identifizieren der betroffenen medizinischen Geräte und ihrer Betreiber
3. Festlegen von Abmilderungs-/Korrekturmaßnahmen
4. Freigabe der FSN
5. Gerätebetreiber per Brief benachrichtigen
6. Betroffene(s) medizinische Gerät(e) ausfindig machen
7. Medizinisches Gerät aus dem klinischen Prozess entfernen
8. Anwenden von Abhilfe-/Korrekturmaßnahmen
9. Medizinisches Gerät wieder einsetzen
10. Beenden

Die Zeitspanne für Schritt 5 (Benachrichtigung des Gerätebetreibers) liegt im Bereich von Tagen. Die Zeitspanne für Schritt 6 (betroffene Geräte ausfindig machen) kann aus verschiedenen Gründen zwischen Stunden und Wochen liegen.

Die Geräteflotte der medizinischen Geräte kann aus vielen Geräten (bis zu mehreren Tausend) bestehen, die über verschiedene Regionen, Städte, Standorte, Gebäude, Räume verteilt sind. Die medizinischen Geräte können auch Teil von Pools sein und verschiedenen Einsatzbereichen dynamisch zugewiesen werden. Die medizinischen Geräte können mobil eingesetzt werden, z. B. für den Patiententransport.

Betroffene medizinische Geräte werden in der Regel anhand ihrer Seriennummer oder einer anderen eindeutigen ID identifiziert, die oft nicht direkt sichtbar am Gerät ist. Solange die betroffenen medizinischen Geräte noch nicht lokalisiert wurden bzw. noch verwendet werden, sind die Patienten und Anwender ggf. einer Exposition ausgesetzt bzw. von den in der FSN hervorgehobenen produktbezogenen Risiken betroffen.

Der Erfindung liegt daher die Aufgabe zugrunde, ein System und Verfahren zur schnellen Risikominderung für Medizinprodukte, die von einer FSN betroffen sind bereitzustellen, welches die Auswirkungen auf Patienten und Benutzer, durch die in der FSN hervorgehobenen produktbezogenen Risiken deutlich reduziert.

In Bezug auf das System wird diese Aufgabe erfindungsgemäß gelöst durch ein System mit den Merkmalen von Anspruch 1. Das System umfasst ein FSN-Modul, welches eine Datenbank umfasst, in welcher wenigstens eine FSN und eindeutige Kennungen von medizinischen Geräten, die von der jeweiligen FSN betroffen sind, sowie ein Katalog, d.h. im Wesentlichen eine Liste, von Maßnahmen zur sofortigen Risikominderung, gespeichert und/oder speicherbar sind. Das System umfasst weiterhin ein Geräteverwaltungsmodul, in welchem eine Liste der betriebenen medizinischen Geräte und jeweils die eindeutige Kennung des jeweiligen medizinischen Geräts hinterlegt ist, und in welchem eine Anzahl von Geräteverwaltungsfunktionen implementiert ist. Das System umfasst weiterhin wenigstens ein medizinisches Gerät, welches eine eindeutige Kennung aufweist, sowie eine Steuereinheit und eine Anzeigeeinheit umfasst.

Das FSN-Modul und das Geräteverwaltungsmodul können in einer bevorzugten Ausführungsform hardware- und/oder softwaremäßig in einer gemeinsamen Computer-Architektur implementiert sein. Beispielsweise können das FSN-Modul und das Geräteverwaltungsmodul jeweils durch Software und einzeln und/oder gemeinsam genutzte Hardware-Komponenten implementiert sein. In einer weiteren bevorzugten Ausführungsform ist das FSN-Modul als eine FSN-Servereinrichtung und das Geräteverwaltungsmodul als eine Geräteverwaltungseinrichtung ausgebildet, welche ausgebildet sind, über ein Datennetz zu kommunizieren und dazu entsprechende Schnittstellen aufweisen.

Das FSN-Servermodul ist dazu ausgebildet, digitale Nachrichten mit dem Geräteverwaltungsmodul und/oder mit dem jeweiligen medizinischen Gerät, insbesondere über ein Datennetz, auszutauschen. Das Geräteverwaltungsmodul ist dazu ausgebildet, digitale Nachrichten mit dem FSN-Modul und/oder mit dem jeweiligen medizinischen Gerät, insbesondere über ein Datennetz, auszutauschen. Das jeweilige medizinische Gerät ist dazu ausgebildet, digitale Nachrichten mit dem FSN-Modul und/oder mit dem Geräteverwaltungsmodul, insbesondere über ein Datennetz, auszutauschen und Maßnahmen zur sofortigen Risikominderung durchzuführen.

Vorteilhafte Ausbildungen der Erfindung sind Gegenstand der Unteransprüche.

Die Erfindung geht von der Überlegung aus, dass die Zeitspanne zwischen der Erstellung bzw. der Freigabe der FSN und der Umsetzung der notwendigen Maßnahmen reduziert werden sollte, sodass nach Bekanntwerden der Risiken diese möglichst schnell in dem jeweiligen medizinischen Gerät durch entsprechende Maßnahmen behandelt werden können.

Wie nunmehr erkannt wurde, erlaubt die Bereitstellung digitaler Informationen über eine neue FSN für Gerätebetreiber und Verteilung von FSN-Informationen an betroffene Medizinprodukte die sofortige Umsetzung risikomindernder Maßnahmen, wie die Anzeige einer Warnmeldung für Betreiber und Anwender, automatische Deaktivierung der betroffenen Gerätefunktionen oder andere Maßnahmen. Die Umsetzung dieser Maßnahmen ist somit eine Frage von Sekunden nach der Genehmigung des FSN, während herkömmliche Methoden zur Ausführung eines FSN in der Regel länger dauern und das Risiko bergen, dass nicht alle betroffenen Geräte erfasst werden.

Vorteilhafterweise weist das Geräteverwaltungsmodul ein Front-End zur Benutzerinteraktion auf. Das Front-End kann zur Ausführung in einem Web-Browser auf einem Desktop-PC und/oder auf einem mobilen Gerät oder in einer nativen App für mobile Geräte ausgebildet sein.

Das Front-End ist vorzugsweise dazu ausgebildet, eine Liste von betriebenen und durch die FSN betroffenen medizinischen Geräten anzuzeigen. Auf diese Weise kann der Geräteverwalter bzw. Benutzer der Geräteverwaltungseinrichtung einen Überblick über die von der FSN betroffenen medizinischen Geräte erhalten.

Vorteilhafterweise ist das Front-End dazu ausgebildet, eine Warnnachricht für den Benutzer auszugeben. Die Warnnachricht umfasst bevorzugt die von der FSN betroffenen Geräte und/oder Informationen der FSN. Auf diese Weise erhält der Benutzer bzw. Gerätebetreuer der Geräteverwaltungseinrichtung eine Übersicht über die Anzahl und den Typ der betroffenen medizinischen Geräte sowie die Art der FSN.

Vorteilhafterweise ist die Anzeigeeinheit ausgebildet ist, bei Vorliegen einer FSN eine Warnnachricht und/oder risikomindernde Maßnahme anzuzeigen.

Das jeweilige medizinische Gerät ist bevorzugt dazu ausgebildet, therapeutische und/oder Patientenüberwachungs- und/oder Diagnosefunktionen auszuführen.

In einer bevorzugten Ausführungsform ist das medizinische Gerät eine Infusionspumpe oder eine Dialysemaschine.

Die eindeutige Kennung des medizinischen Gerätes ist vorteilhafterweise eine UDID.

In einer bevorzugten Ausführungsform werden die digitalen Daten über ein Datennetz ausgetauscht, wobei das Datennetz vorteilhafterweise das Internet ist. Dadurch können Daten zwischen den einzelnen Systemkomponenten (FSN-Modul, Geräteverwaltungsmodul, medizinisches Gerät) über beliebige Entfernungen ausgetauscht werden. Es erlaubt das Aufstellen der Einrichtungen bzw. Hosten der zugehörigen Daten an unterschiedlichen Standorten. Das FSN-Modul, das Geräteverwaltungsmodul und das jeweilige medizinische Gerät weisen dazu jeweils eine entsprechende Schnittstelle auf, insbesondere eine kabelgebundene Netzwerkschnittstelle und/oder eine WLAN-Schnittstelle.

Das FSN-Modul weist bevorzugt eine Push-Funktion für eine FSN auf, sodass nach erfolgter Genehmigung der FSN diese an das Geräteverwaltungsmodul bzw. die betroffenen medizinischen Geräte gepusht werden kann. Das Geräteverwaltungsmodul und/oder das jeweilige medizinische Gerät kann auch bevorzugt eine Pull-Funktion aufweisen, mit deren Hilfe in regelmäßigen Abständen das FSN-Modul kontaktiert wird und eine genehmigte FSN heruntergeladen werden kann.

Das Geräteverwaltungsmodul und/oder das jeweilige medizinische Gerät ist vorteilhafterweise dazu ausgebildet, bei Erhalt einer FSN eine Empfangsbestätigung an das FSN-Modul zu senden. Auf diese Weise kann sichergestellt werden, dass die FSN und die Maßnahmen zur sofortigen Risikominderung das medizinische Gerät erreicht haben.

Das jeweilige medizinische Gerät ist bevorzugt dazu ausgebildet, nach Umsetzung der Maßnahmen zur sofortigen Risikominderung eine Umsetzungsbestätigung an das Geräteverwaltungsmodul und/oder das FSN-Modul zu senden bzw. eine Umsetzungsbestätigung auf einer Benutzerschnittstelle, insbesondere einem Display, anzuzeigen.

In einer bevorzugten Ausführungsform ist das Geräteverwaltungsmodul dazu ausgebildet, den Status der zu einer FSN gehörenden Umsetzung der Maßnahmen zur sofortigen Risikominderung für das jeweilige medizinisches Gerät abzuspeichern.

In Bezug auf das computerimplementierte Verfahren wird die oben genannte Aufgabe erfindungsgemäß gelöst durch ein Verfahren mit den Merkmalen von Anspruch 8. Das Verfahren weist die unten genannten Schritte auf, wobei die Reihenfolge der Verfahrensschritte durch die unten angegebene Reihenfolge der Schritte nicht bestimmt ist:
- in dem FSN-Modul wird bevorzugt eine neue FSN registriert bzw. gespeichert. Dazu werden bevorzugt in das FSN-Modul die für die FSN relevanten Informationen eingegeben: eine FSN-Kennung, durch welche die FSN eindeutig identifizierbar ist, eine Liste der betroffenen Geräte bzw. deren eindeutige Kennungen, Daten zur Beschreibung der risikomindernden Maßnahmen (englisch IRMCD - Instant Risk Mitigation Control Data).
- in dem FSN-Modul werden bevorzugt die von der FSN betroffenen medizinischen Geräte hinterlegt. Die Identifizierung der betroffenen Geräte erfolgt bevorzugt außerhalb des beschriebenen Systems in einem übergeordneten FSN-Prozess durch einen Sicherheitsbeauftragten. In der FSN-Servereinrichtung wird durch Eingabe von Daten die Verbindung zwischen FSN und betroffenen Geräten hergestellt.
- in dem FSN-Modul werden bevorzugt die Maßnahmen zur sofortigen Risikominderung festgelegt.
- in dem FSN-Modul wird vorteilhafterweise die FSN freigegeben. Die Freigabe der FSN ist Teil des übergeordneten FSN Prozesses und wird in dem FSN-Modul durch eine entsprechende Aktion, zum Beispiel durch eine Statusänderung digital nachgebildet.
- das FSN-Modul sendet bevorzugt eine digitale Nachricht an das Geräteverwaltungsmodul, umfassend eine Liste von Daten zu den von der FSN betroffenen medizinischen Geräte.
   - -das Geräteverwaltungsmodul sendet vorteilhafterweise an alle betroffenen medizinischen Geräte, welche verbunden und online sind, eine FSN-Nachricht, welche die Maßnahmen zur sofortigen Risikominderung enthält.
- das jeweilige betroffene medizinische Gerät führt bevorzugt die Maßnahmen zur sofortigen Risikominderung durch.

Die Maßnahmen zur sofortigen Risikominderung sind bevorzugt abmildernde und/oder korrigierende Maßnahmen.

Die Maßnahmen zur sofortigen Risikominderung können die Ausgabe einer Warnnachricht an den Benutzer mit einer Beschreibung der FSN, insbesondere der darin beschriebenen Risiken für Patienten und/oder Anwender umfassen. Es kann auch ein maschinenlesbarer Code, beispielsweise ein QR-Code ausgegeben werden, welcher einen Link bzw. einen Verweis zu vom Benutzer durchzuführenden Maßnahmen zur sofortigen Risikominderung enthält.

Ein weiteres Beispiel für eine Risikomindernde Maßnahme ist die Deaktivierung oder Einschränkung einer Gerätefunktion, die das Risiko hervorruft, beispielsweise die Reduzierung der Hintergrundbeleuchtung eines Displays, um den kompletten Ausfall der Displayeinheit bei zu hohen Leuchtstärken zu vermeiden.

In einer bevorzugten Ausführungsform zeigt das von der FSN betroffene medizinische Gerät nach Erhalt der FSN-Nachricht auf der Anzeigeeinheit eine Warnnachricht an.

Vorteilhafterweise benachrichtigt das jeweilige medizinische Gerät das Geräteverwaltungsmodul über die erfolgte Durchführung der abmildernden/korrigierenden Maßnahmen.

Bevorzugt wird nach der Durchführung der abmildernden/korrigierenden Maßnahmen das jeweilige medizinische Gerät wieder in den Einsatz gebracht.

In einer bevorzugten Ausführungsform erstellt das FSN-Modul einen Statusreport zu den durchgeführten Maßnahmen. Auf diese Weise kann zu einem späteren Zeitpunkt nachvollzogen, welche Maßnahmen durchgeführt wurden. Der Statusreport umfasst bevorzugt einen Zeitstempel bzw. ein Zeitintervall, die Art der durchgeführten Maßnahmen und die entsprechende eindeutige Kennung des medizinischen Geräts.

Die Liste von Daten umfasst vorteilhafterweise: eine eindeutige Kennung der FSN, eine Liste der eindeutigen Kennungen der medizinischen Geräte und die Daten zu den Maßnahmen zur sofortigen Risikominderung.

In einer bevorzugten Ausführungsform des Verfahrens gibt nach Erhalt der digitalen Nachricht das Geräteverwaltungsmodul eine Warnnachricht, insbesondere als optische und/oder akustische Warnnachricht und/oder als E-Mail-Nachricht, aus. Auf diese Weise erhält der Benutzer bzw. Betreiber des Geräteverwaltungsmoduls umfassende Informationen über die von der FSN betroffenen medizinischen Geräte und der bevorstehenden Maßnahmen zur sofortigen Risikominderung. Die Nachricht beschreibt bevorzugt die FSN und zeigt eine Liste aller betroffenen medizinischen Geräte an.

Vorteilhafterweise verbinden sich medizinische Geräte, welche nicht bei dem Versenden der FSN-Nachricht verbunden oder online waren, mit dem Geräteverwaltungsmodul, um das Vorhandensein einer FSN-Nachricht abfragen und diese zu empfangen (sofern sie vorhanden ist).

Das Verbinden mit dem Geräteverwaltungsmodul erfolgt bevorzugt aufgrund einer Triggerbedingung, insbesondere bei Einschalten des Geräts, Aktivierung bestimmter Gerätefunktionen, Aktivierung der Netzschnittstelle, bei einer bestimmten Tageszeit, aufgrund eines zyklischen Timers. Dadurch wird sichergestellt, dass ein Abfragen der FSN-Nachricht zuverlässig erfolgt und FSN-Nachrichten von dem medizinischen Gerät zeitnah abgefragt werden.

Vorteilhafterweise werden medizinische Geräte, bei welchen eine Reparatur durchgeführt werden muss, außer Betrieb genommen.

Die FSN-Nachricht umfasst vorteilhafterweise weitere Maßnahmen, insbesondere Reparaturmaßnahmen.

Die Daten zu den Maßnahmen zur sofortigen Risikominderung, insbesondere abmildernde/korrigierende Maßnahmen, sind bevorzugt Daten in der FSN-Nachricht enthalten.

Die Vorteile der Erfindung liegen insbesondere darin, dass durch Ausbildung der beteiligten Einrichtungen bzw. Geräte und ihre Kommunikation über ein Datennetz die Zeitspanne zwischen der Erstellung einer FSN und dem Beginn der Umsetzung der entsprechenden Maßnahmen zur sofortigen Risikominderung nur wenige Sekunden beträgt, da die von der FSN betroffenen medizinischen Geräte unmittelbar nach der Erstellung der FSN über das Datennetz benachrichtigt werden. Dadurch wird die Zeit, in welcher Patienten oder Benutzer den in der FSN angezeigten Risiken weiter ausgesetzt sind, stark reduziert.

Ein Ausführungsbeispiel der Erfindung wird nachfolgend anhand einer Zeichnung näher erläutert. Darin zeigen in stark schematisierter Darstellung:
- FIG. 1: ein System zur sofortigen Risikominderung in einer bevorzugten Ausführungsform, und
- FIG. 2: ein Verfahren zum Betreiben des Systems gemäß FIG. 1 in einem Ablaufdiagramm in einer bevorzugten Ausführungsform.

Gleiche Teile oder Schritte sind in beiden Figuren mit denselben Bezugszeichen versehen.

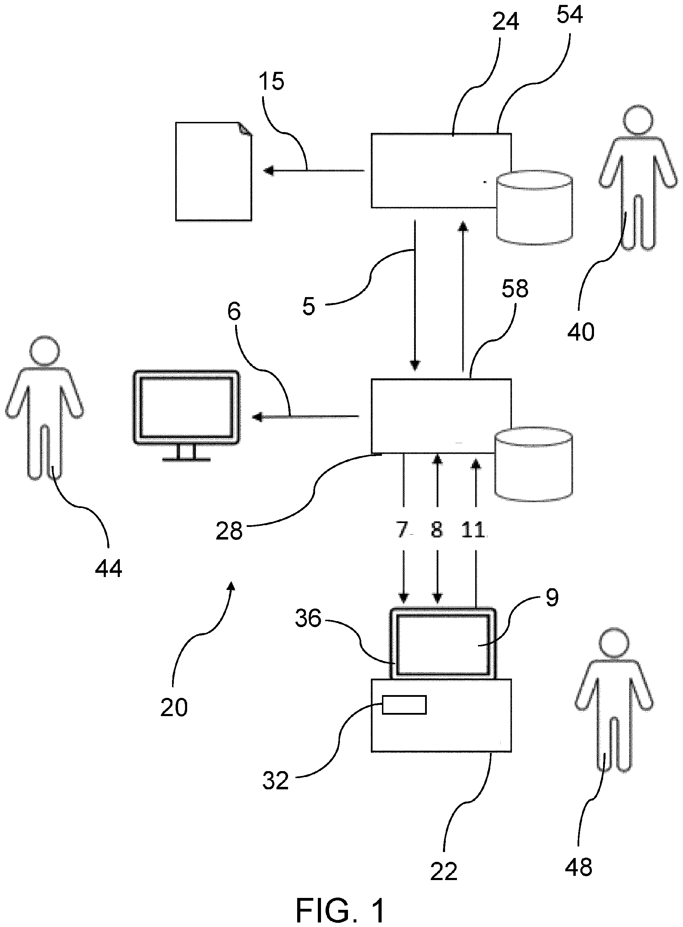

Das in FIG. 1 dargestellte System 20 umfasst ein FSN-Modul 54, welches vorliegend als eine FSN-Servereinrichtung 24 ausgebildet ist. Die FSN-Servereinrichtung 24 umfasst eine Softwareanwendung und/oder einen Dienst, der eine Datenbank mit gerätebezogenen FSN unterhält. Die FSN-Servereinrichtung 24 wird bevorzugt vom rechtmäßigen Hersteller eines medizinischen Geräts 22 gehostet und umfasst für jede FSN eine Liste der betroffenen medizinischen Geräte 22, die durch ihre eindeutige Kennung, insbesondere Geräte-ID (UDID), identifiziert werden. Der Übersichtlichkeit halber ist in FIG. 1 nur ein einziges medizinisches Gerät 22 eingezeichnet. Die FSN-Servereinrichtung 24 wird von einem Sicherheitsbeauftragten 40 bedient, insbesondere konfiguriert.

Die FSN-Servereinrichtung 24 umfasst für jede FSN einen Katalog von Daten bzw. Kontrolldaten zur sofortigen Risikominderung (IRMCD), die Maßnahmen zur sofortigen Risikominderung, z. B. Benutzerwarnmeldungen und betroffene Gerätefunktionen, definiert und/oder beschreibt.

Die FSN-Servereinrichtung 24 ist dazu ausgebildet, digitale Nachrichten mit einem Geräteverwaltungsmodul 58, welches vorliegend als Geräteverwaltungseinrichtung 28 ausgebildet ist und/oder dem medizinischen Gerät 22 über ein Datennetz, vorliegend das Internet, auszutauschen. Die FSN-Servereinrichtung 24 und die Geräteverwaltungseinrichtung 28 und das jeweilige medizinische Gerät 22 sind entsprechend für den Datenaustausch über das Internet ausgebildet und weisen entsprechende Schnittstellen auf. Diese Schnittstellen können insbesondere kabelgebundene oder drahtlose Schnittstellen, insbesondere WLAN, sein.

Das System 20 umfasst die oben genannte Geräteverwaltungseinrichtung 28, welche eine Softwareanwendung oder einen Dienst umfasst und welche eine Liste der eingesetzten medizinischen Geräte 22 verwaltet. Die Geräteverwaltungseinrichtung 28 ist dazu ausgebildet, digitale Nachrichten mit der FSN-Servereinrichtung 24 und/oder dem jeweiligen medizinischen Gerät 22 über das Datennetz bzw. Netzwerk auszutauschen. Das jeweilige medizinische Gerät 22 weist eine UDID (Seriennummer oder eine andere eindeutige ID) auf. Die Geräteverwaltungseinrichtung 28 speichert für jedes medizinische Gerät 22 die zugehörige UDID.

Die Geräteverwaltungseinrichtung 28 wird entweder vor Ort (z. B. in der IT-Landschaft des Betreibers) oder in einer Cloud gehostet. Sie umfasst eine Front-end-Anwendung, die in Webbrowsern oder auf mobilen Geräten läuft und einem Gerätebetreuer 44 das Abrufen von Informationen und/oder Interaktionen ermöglicht.

Die Geräteverwaltungseinrichtung 28 zeigt bedarfsweise eine Liste der medizinischen Geräte 22 an, die vom Betreiber eingesetzt und verwendet werden. Sie bietet verschiedene Geräteverwaltungsfunktionen an, wie beispielsweise die Anzeige des Gerätestatus, das Hochladen von Gerätekonfigurationsdaten usw.

Das System 20 umfasst das wenigstens eine medizinisches Gerät 22 mit therapeutischen, Patientenüberwachungs- oder Diagnosefunktionen. Das jeweilige medizinische Gerät 22 ist softwaregesteuert, einschließlich eines oder mehrerer Mikroprozessoren zur Ausführung dieser Software.

Das jeweilige medizinische Gerät 22 umfasst weiterhin eine Steuereinheit 32 und eine Anzeigeeinheit 36 für die Benutzerinteraktion (Benutzereingabe, grafische Ausgabe) für einen Gerätebenutzer 48. Das medizinische Gerät 22 ist dazu ausgebildet, digitale Nachrichten mit der FSN-Servereinrichtung 24 und/oder der Geräteverwaltungseinrichtung 28 über das Datennetz auszutauschen.

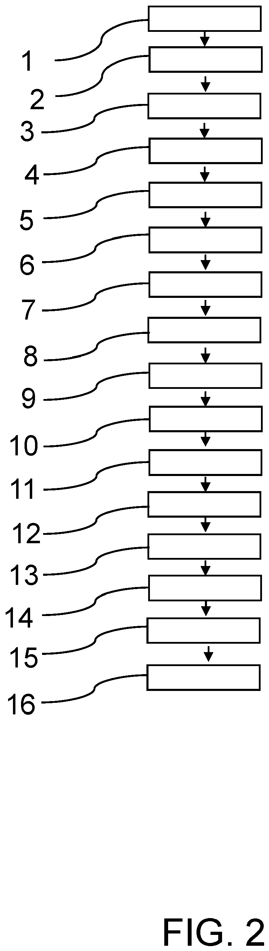

Anhand der FIG. 1 und der FIG. 2 werden im Folgenden die Funktionalitäten des Systems 20 und ein Verfahren zum Betreiben des Systems 20 in einer bevorzugten Ausführungsform erläutert. In der FIG. 2 ist das Verfahren in einem Ablaufdiagramm dargestellt. Zur Erläuterung sind einige Schritte des Verfahrens in FIG. 1 durch Bezugszeichen kenntlich gemacht.

Das computerimplementierte Verfahren umfasst dabei die im Folgenden beschriebenen Schritte.

In einem ersten Schritt 1 wird in der FSN-Servereinrichtung 24 eine neue FSN registriert bzw. gespeichert.

In einem zweiten Schritt 2 werden in der FSN-Serverreinrichtung 24 die von der FSN betroffenen medizinischen Geräte 22 hinterlegt bzw. gespeichert.

In einem dritte Schritt 3 werden in der FSN-Servereinrichtung die Maßnahmen zur sofortigen Risikominderung festgelegt.

In einem vierten Schritt 4 wird in der FSN-Servereinrichtung 24 die FSN freigegeben bzw. genehmigt. Die Schritte 1-4 werden bevorzugt von einem Benutzer, insbesondere dem Sicherheitsbeauftragten 40, durchgeführt.

In einem fünften Schritt 5 lädt die FSN-Servereinrichtung 24 die Liste der eindeutigen Kennungen der betroffenen medizinischen Geräte 22 und die Daten zu Maßnahmen zur sofortigen Risikominderung in die Geräteverwaltungseinrichtung 28 hoch.

In einem sechsten Schritt 6 benachrichtigt die Geräteverwaltungseinrichtung 28 den Gerätebetreiber durch eine Benachrichtigung über eine neue FSN und die betroffenen medizinischen Geräte 22. Diese Benachrichtigung bzw. Warnung kann auf verschiedene Weise implementiert werden, z. B. durch Anzeige einer Meldung und/oder einer Liste der betroffenen medizinischen Geräte 22 im Front-End der Geräteverwaltungseinrichtung 28, Senden einer E-Mail-Benachrichtigung an den Betreiber, usw.

In einem siebten Schritt 7 sendet die Geräteverwaltungseinrichtung 28 eine FSN-Nachricht einschließlich der Daten zu den Maßnahmen zur sofortigen Risikominderung (IRMCD) an alle betroffenen medizinischen Geräte 22, die verbunden und online sind.

In einem achten Schritt 8 fragen die medizinischen Geräte 22, welche vorübergehend deaktiviert oder nicht mit dem Netzwerk verbunden sind, während die Geräteverwaltungsrichtung 28 die FSN-Daten sendet, nach FSN-Ereignissen ab, wenn sie wieder online sind. Die medizinischen Geräte 22 können auch andere Auslöser für die Abfrage von FSN-Ereignissen verwenden, wie z. B. Start des medizinischen Geräts 22, Aktivierung definierter Gerätefunktionen, Aktivierung der Netzwerkschnittstelle, definierte Tageszeit, zyklischer Timer.

In einem neunten Schritt 9 benachrichtigt das betroffene medizinische Gerät 22 den Gerätebenutzer über eine neue FSN, indem es eine Warnmeldung auf dem Display bzw. der Anzeigeeinheit 36 des medizinischen Geräts 22 anzeigt, einschließlich Benutzerführung für die weitere Handhabung des medizinischen Geräts 22. Die Meldungstexte, welche bevorzugt Bestandteil der Daten zu den Maßnahmen zur sofortigen Risikominderung sind, können bevorzugt vom Sicherheitsbeauftragen 40 angepasst werden
In einem zehnten Schritt 10 führt das Gerät andere unmittelbare risikomindernde Maßnahmen aus, z. B. die Deaktivierung der Funktionen der betroffenen medizinischen Geräte 22.

In einem elften Schritt 11 bestätigt das medizinische Gerät 22 den Empfang des FSN-Ereignisses an die Geräteverwaltungseinrichtung 28, einschließlich der Bestätigung der durchgeführten Risikominderungsmaßnahmen. Die Geräteverwaltungseinrichtung 28 leitet es dann an die FSN-Servereinrichtung weiter 24.

In einem zwölften Schritt 12 wird das medizinische Gerät 22 aus dem klinischen Prozess entfernt, falls eine physische Reparatur des medizinischen Geräts 22 erforderlich ist.

In einem dreizehnten Schritt 13 werden weitere Maßnahmen zur sofortigen Risikominderung, d.h. Abmilderungs-/Korrekturmaßnahmen, gemäß FSN, durchgeführt, z. B. eine Gerätereparatur.

In einem vierzehnten Schritt 14 wird das medizinische Gerät 22 wieder eingesetzt, d.h. in Betrieb genommen.

In einem fünfzehnten Schritt 15 erstellt die FSN-Servereinrichtung 24 einen Statusbericht über die durchgeführten Abhilfemaßnahmen; dies entspricht einer gesetzlichen Anforderung.

In einem sechzehnten Schritt 16 wird das Verfahren beendet.

Im Gegensatz zu dem oben beschriebenen Verfahren nach dem Stand der Technik vergehen zwischen Schritt 4 (FSN genehmigen) und den Schritten 9 und 10, in denen sofortige Maßnahmen zur Risikominderung ergriffen werden, nur wenige Sekunden für die Gesamtheit der betroffenen Geräte, wodurch die Exposition der Patienten und/oder Anwender gegenüber den in der FSN hervorgehobenen gerätebezogenen Risiken gestoppt wird. Geräte, die bei der anfänglichen Eskalation des FSN-Ereignisses übersehen wurden, werden immer den achten Schritt 8 verwenden, um die neuesten FSN-Daten zu erhalten, wenn sie wieder online sind.

### Bezugszeichenliste

- 1: erster Schritt
- 2: zweiter Schritt
- 3: dritter Schritt
- 4: vierter Schritt
- 5: fünfter Schritt
- 6: sechster Schritt
- 7: siebter Schritt
- 8: achter Schritt
- 9: neunter Schritt
- 10: zehnter Schritt
- 11: elfter Schritt
- 12: zwölfter Schritt
- 13: dreizehnter Schritt
- 14: vierzehnter Schritt
- 15: fünfzehnter Schritt
- 16: sechzehnter Schritt
- 22: medizinisches Gerät
- 20: System
- 24: FSN-Servereinrichtung
- 28: Geräteverwaltungseinrichtung
- 32: Steuereinheit
- 36: Anzeigeeinheit
- 40: Sicherheitsbeauftragter
- 44: Gerätebetreuer
- 48: Gerätebenutzer
- 54: FSN-Modul
- 58: Geräteverwaltungsmodul

## Patentansprüche

1. System (20) zur sofortigen Risikominderung für medizinische Geräte (22), die von einer FSN betroffen sind, umfassend
- ein FSN-Modul (54), welches eine Datenbank aufweist, in welcher wenigstens eine FSN und eindeutige Kennungen von medizinischen Geräten (22), die von der jeweiligen FSN betroffen sind, sowie ein Katalog von Daten zu Maßnahmen zur sofortigen Risikominderung für die jeweilige FSN gespeichert sind und/oder speicherbar sind;
- ein Geräteverwaltungsmodul (58), in welchem eine Liste von medizinischen Geräten (22) im Einsatz und jeweils die eindeutige Kennung des jeweiligen medizinischen Geräts (22) hinterlegt ist, und in welchem eine Anzahl von Geräteverwaltungsfunktionen implementiert ist;
- wenigstens ein medizinisches Gerät (22), welches eine eindeutige Kennung aufweist, und eine Steuereinheit (32) und eine Anzeigeeinheit (36) umfasst, wobei
• das FSN-Modul (54) dazu ausgebildet ist, digitale Nachrichten mit dem Geräteverwaltungsmodul (58) und/oder mit dem jeweiligen medizinischen Gerät (22), insbesondere über ein Datennetz, auszutauschen, und wobei
• das Geräteverwaltungsmodul (58) dazu ausgebildet ist, digitale Nachrichten mit dem FSN-Modul (54) und/oder mit dem jeweiligen medizinischen Gerät (22), insbesondere über ein Datennetz, auszutauschen, und wobei
• das jeweilige medizinische Gerät (22) dazu ausgebildet ist, digitale Nachrichten mit dem FSN-Modul (54) und/oder mit dem Geräteverwaltungsmodul (58), insbesondere über ein Datennetz, auszutauschen und Maßnahmen zur sofortigen Risikominderung durchzuführen.

2. System (20) nach Anspruch 1, wobei das Geräteverwaltungsmodul (58) ein Front-End zur Benutzerinteraktion aufweist.

3. System (20) nach Anspruch 2, wobei das Front-End ausgebildet ist, eine Liste von betriebenen und durch die FSN betroffenen medizinischen Geräten (22) anzuzeigen und/oder wobei das Front-End ausgebildet ist, eine Warnnachricht für den Benutzer auszugeben.

4. System (20) nach einem der Ansprüche 1 bis 3, wobei die Anzeigeeinheit (36) ausgebildet ist, bei Vorliegen einer FSN eine Warnnachricht und/oder risikomindernde Maßnahme anzuzeigen.

5. System (20) nach einem der Ansprüche 1 bis 4, wobei das jeweilige medizinische Gerät (22) ausgebildet ist, therapeutische und/oder Patientenüberwachungs- und/oder Diagnosefunktionen auszuführen.

6. System (20) nach einem der vorherigen Ansprüche, wobei das medizinische Gerät eine Infusionspumpe oder eine Dialysemaschine ist.

7. System (20) nach einem der vorherigen Ansprüche, wobei die digitalen Daten über ein Datennetz ausgetauscht werden, und wobei das Datennetz das Internet ist.

8. Computerimplementiertes Verfahren zum Betreiben eines Systems (20) nach einem der vorherigen Ansprüche, mit den folgenden Schritten:
- in dem FSN-Modul (54) wird eine neue FSN registriert;
- in dem FSN-Modul (54) werden die von der FSN betroffenen medizinischen Geräte (22) hinterlegt;
- in dem FSN-Modul (54) werden die Maßnahmen zur sofortigen Risikominderung festgelegt;
- in dem FSN-Modul (54) wird die FSN freigegeben;
- das FSN-Modul (54) schickt eine digitale Nachricht an das Geräteverwaltungsmodul (58), umfassend eine Liste von Daten zu den von der FSN betroffenen medizinischen Geräten (22);
- das Geräteverwaltungsmodul (58) sendet an alle betroffenen medizinischen Geräte (22), welche verbunden und online sind, eine FSN-Nachricht, welche die Daten zu den Maßnahmen zur sofortigen Risikominderung enthält;
- von der FSN betroffene medizinische Geräte (22) zeigen optional nach Erhalt der FSN-Nachricht auf der Anzeigeeinheit (36) eine Warnnachricht;
- das jeweilige betroffene medizinische Gerät (22) führt die Maßnahmen zur sofortigen Risikominderung durch;
- das jeweilige medizinische Gerät (22) benachrichtigt optional das Geräteverwaltungsmodul (58) über die erfolge Durchführung der Maßnahmen zur sofortigen Risikominderung.

9. Verfahren nach Anspruch 8, wobei die Liste von Daten umfasst: eine eindeutige Kennung der FSN, eine Liste der eindeutigen Kennung der medizinischen Geräte (22) und die Daten zu Maßnahmen zur sofortigen Risikominderung.

10. Verfahren nach Anspruch 8 oder 9, wobei in nach Erhalt der digitalen Nachricht das Geräteverwaltungsmodul (58) eine Warnnachricht ausgibt, insbesondere als optische und/oder akustische Warnnachricht und/oder als E-Mail-Nachricht.

11. Verfahren nach einem der Ansprüche 8 bis 10, wobei medizinische Geräte (22), welche nicht bei dem Versenden der FSN-Nachricht verbunden oder online waren, sich mit dem Geräteverwaltungsmodul (58) verbinden, um das Vorhandensein einer FSN-Nachricht abfragen und diese zu empfangen.

12. Verfahren nach Anspruch 11, wobei das Verbinden mit dem Geräteverwaltungsmodul (58) aufgrund einer Triggerbedingung erfolgt, insbesondere bei Einschalten des Geräts, Aktivierung bestimmter Gerätefunktionen, Aktivierung der Netzschnittstelle, bei einer bestimmten Tageszeit, aufgrund eines zyklischen Timers.

13. Verfahren nach einem der Ansprüche 8 bis 12, wobei in einem Schritt 12) medizinische Geräte (22), bei welchen eine Reparatur durchgeführt werden muss, außer Betrieb genommen werden.

14. Verfahren nach einem der Ansprüche 8 bis 13, wobei die FSN-Nachricht weitere Maßnahmen, insbesondere Reparaturmaßnahmen, enthält.

15. Verfahren nach einem der Ansprüche 8 bis 14, wobei die Daten für die Maßnahmen zur sofortigen Risikominderung als Instant Risk Mitigation Control Data in der FSN-Nachricht enthalten sind.
